# EUROPÄISCHE PATENTSCHRIFT

(11) **EP 0 717 976 B1**
(45) Veröffentlichungstag und Bekanntmachung des Hinweises auf die Patenterteilung: **03.09.2003**
(21) Anmeldenummer: 95119769.8
(22) Anmeldetag: 15.12.1995
(51) Int. Cl.: A61K 6/083

(54) **Polymerisierbares Dentalmaterial**
Polymerisable dental material
Matériau dentaire polymérisable

(30) Priorität: 23.12.1994 DE 4446033
(43) Veröffentlichungstag der Anmeldung: 26.06.1996
(73) Patentinhaber: Heraeus Kulzer GmbH & Co.KG, 63450 Hanau (DE)
(72) Erfinder: Erdrich, Albert, Dr., D-61231 Bad Nauheim (DE); Eck, Michael, D-61389 Schmitten (DE); Reischl, Kurt, D-35799 Merenberg 2 (DE); Weber-Pelka, Slawomira, D-61352 Bad Homburg (DE)
(74) Vertreter: Kühn, Hans-Christian

(56) Entgegenhaltungen:
- DE-A- 2 405 578
- DE-A- 2 446 546
- DE-A- 3 400 130
- US-A- 4 536 523

## Beschreibung

Die Erfindung betrifft ein polymerisierbares Dentalmaterial, das monomere (Meth)Acrylsäureester, feinteiligen anorganischen Füllstoff und einen Polymerisationskatalysator enthält.

Das erfindungsgemäße Dentalmaterial - ein Komposit - eignet sich besonders zur Herstellung von Zahnfüllungen, Kronen, Brücken, Verblendungen, Inlays, Onlays und künstlichen Zähnen sowie als Befestigungszement.

Polymerisierbare Dentalmaterialien zur Herstellung von Zahnfüllungen sind seit vielen Jahren bekannt. Die ersten dieser Materialien bestanden aus Mischungen von monomerem mit polymerem Methylmethacrylat, die durch Zusatz eines Katalysators bzw. eines Systems aus Katalysator und Beschleuniger innerhalb weniger Minuten unter den im Munde herrschenden Bedingungen aushärteten.

Eine Verbesserung der mechanischen Eigenschaften dieser Dentalmaterialien wurde durch den Zusatz von feinteiligen Füllstoffen, wie Quarz oder Aluminiumsilicaten, eine Verbesserung der ästhetischen Wirkung durch die Entwicklung neuer Katalysator-Systeme, die keine Verfärbung mehr verursachen, und eine Verringerung der Polymerisationsschrumpfung durch die Verwendung von Methacrylsäureestern höherer Alkohole neben oder anstelle von Methylmethacrylat erreicht.

Das erste dieser neuen Materialien wurde von Rafael L. Bowen entwickelt und in der US-Patentschrift 3 066 112 beschrieben. Es enthält als monomeres Bindemittel im wesentlichen ein durch Reaktion eines Bis-Phenols mit Glycidylacrylat beziehungsweise -methacrylat dargestelltes Diacrylat oder Dimethacrylat und als anorganischen Füllstoff feinteiliges Siliciumdioxid, vorzugsweise in silanisierter Form. Das von Bowen gefundene Bis-[4-(2-hydroxy-3-methacryloyloxypropoxy)-phenyl]-dimethylmethan, auch als Bis-GMA oder Bowen-Monomer bezeichnet, ist heute in den meisten der im Handel befindlichen Dentalmaterialien enthalten.

Ein Beispiel für ein weiteres Komposit - ein Dentalmaterial, das neben organischen Monomeren einen feinteiligen anorganischen Füllstoff enthält - wird in der US-Patentschrift 3 539 533 beschrieben. Das polymerisierbare Bindemittel ist dabei ein Gemisch aus Bis-GMA, Bisphenol A-dimethacrylat, verdünnendem Monomer, besonders Triethylenglykoldimethacrylat, und gegebenenfalls Methacrylsäure in geringer Menge, das zusammen mit etwa 65 - 75 Gewichts-% des anorganischen Füllstoffs, zum Beispiel aus Siliciumdioxid, Glas, Aluminiumoxid oder Quarz, verwendet wird. Der anorganische Füllstoff kann eine Teilchengröße von etwa 2 - 85 Mikrometer besitzen; zur Verbesserung des Verbundes Kunststoff/Füllstoff wird er mit einem Silan, wie zum Beispiel 3-Methacryloyloxypropyltrimethoxysilan, vorbehandelt.

Aus DE 24 03 211 C3 ist ein Werkstoff für Dentalzwecke (Füllungsmaterialien für Kavitäten, Materialien für Befestigungszemente, Versiegelungs- und Schutzüberzugsmassen, Kronen- und Brückenmaterialien, Prothesenmaterialien, Massen zur Herstellung künstlicher Zähne) bekannt, der neben polymerisierbarem Acrylat beziehungsweise Methacrylat als anorganischen Füllstoff mikrofeines (hochdisperses) Siliciumdioxid mit einer Teilchengröße von etwa 0,01 - 0,4 Mikrometer enthält. Das polymerisierbare Monomer besteht dabei aus Bis-GMA oder einem anderen Derivat des Bisphenol A oder einem Reaktionsprodukt aus Hydroxyalkylmethacrylaten und Diisocyanaten, gegebenenfalls zusammen mit monomeren kurzkettigen Methacrylaten und/oder Diacrylaten beziehungsweise Dimethacrylaten. Die aus dem den mikrofeinen Füllstoff enthaltenden Werkstoff hergestellten Zahnfüllungen und dergleichen zeichnen sich durch ihre Hochglanzpolierbarkeit und durch eine der Transparenz natürlicher Zähne ähnliche Transparenz aus.

Aus DE 24 05 578 A1 ist es bekannt, in einem zu hochglanzpolierbaren Produkten zu verarbeitenden Dentalwerkstoff neben Estern der Methacrylsäure als anorganischen Füllstoff durch Fällung oder Flammhydrolyse hergestellte amorphe Kieselsäure mit einer maximalen Teilchengröße von 0,07 Mikrometer, gegebenenfalls im Gemisch mit feinteiligem Glas, dessen Teilchengröße 5 Mikrometer nicht überschreiten sollte, zu verwenden. Als Methacrylsäureester werden darin Bis-GMA, 2,2-Bis-[p-(2-hydroxyethoxy)-phenyl]-propandimethacrylat und Triethylenglykoldimethacrylat genannt.

Ein sowohl konventionelle als auch mikrofeine anorganische Füllstoffe enthaltendes Dentalmaterial - dafür hat sich die Bezeichnung Hybrid-Komposit eingebürgert - wird zum Beispiel auch in der internationalen Patentanmeldung WO 81/02 254 beschrieben. Es enthält ein Füllstoff-Gemisch aus hydrophobem Siliciumdioxid mit einem Durchmesser von 0,01 - 0,04 Mikrometer und Glas, zum Beispiel röntgenopakes barium- oder strontiumhaltiges Glas, mit einem Durchmesser von 2 - 30 Mikrometer. Als polymerisierbare Monomere dienen Bis-GMA oder ethoxyliertes Bisphenol A-dimethacrylat und Triethylenglykoldimethacrylat. Das Material wird als Zahnfüllungsmaterial und zum Verblenden von zum Beispiel gegossenen Gold-Kronen verwendet.

Nach DE 35 32 997 A1 lassen sich Komposite für die Dentalpraxis dadurch herstellen, daß kugelförmige Kieselsäurepartikel, die durch Ultraschallvernebelung von Kieselsolen erhalten werden, in zur Polymerbildung geeignete Mischungen eingebracht werden. Die einen Durchmesser zwischen 0,1 und 4 Mikrometer aufweisenden und gegebenenfalls mit einem Silanhaftmittel modifizierten Kieselsäurepartikel können auch in Kombination mit anderen anorganischen Füllstoffen eingesetzt werden. Die Komposite eignen sich besonders als Kronen- und Brückenmaterial und als kalthärtendes Zahnfüllungsmaterial anstelle von Amalgam. Die besonderen Eigenschaften des Komposits sind:
gute Verarbeitbarkeit und leichte Modellierbarkeit dentaler Werkstücke auch bei Füllstoff-Konzentrationen > 70 %,
geringer Thixotropie-Effekt durch den Zusatz der kugelförmigen Kieselsäure im Vergleich zur amorphen Kieselsäure,
vollständige klebfreie Aushärtung durch Bestrahlung auch bei Schichtdicken > 2 mm,
gleich große Mikrohärte-Werte auf der Ober- und Unterseite des Werkstücks,
Herstellung von hochglänzenden, glatten Oberflächen.

DE 37 08 618 C2 und 38 26 233 C2 betreffen Kunststoff-Zahnersatzteile mit einem abrasionsfesten, hochglanzpolierbaren Mantel aus 10 - 90 Gewichts-% hochdisperses Siliciumdioxid mit einer Teilchengröße von 0,01 - 0,4 Mikrometer enthaltendem Kunststoff. Der Mantel umhüllt einen eine hohe Biegefestigkeit und einen hohen Biegemodul aufweisenden Kern, der 30 - 90 Gewichts-% eines anorganischen Füllstoff-Gemisches aus 60 - 100 Gewichts-% Siliciumdioxid, Lithiumaluminiumsilicat-Glas und/oder Strontiumaluminiumsilicat-Glas mit einer mittleren Teilchengröße von 0,7 - 5 Mikrometer oder Bariumaluminiumsilicat-Glas mit einer mittleren Teilchengröße von 0,7 10 Mikrometer und 0 - 40 Gewichts-% hochdisperses Siliciumdioxid mit einer mittleren Teilchengröße von 0,01 - 0,4 Mikrometer enthält. Der Kunststoff von Kern und Mantel ist vorzugsweise ein Polymer aus Bis-GMA, ethoxyliertem Bisphenol A-diacrylat beziehungsweise -dimethacrylat, Triethylenglykoldimethacrylat, Dodecandioldimethacrylat, Diurethandimethacrylat aus 2-Hydroxyethylmethacrylat und 2,2,4-Trimethylhexamethylendiisocyanat, Bis-(acryloyloxymethyl)-tricyclo-[5.2.1.0^{2,6}]decan und/oder Bis-(methacryloyloxymethyl)-tricyclo[5.2.1.0^{2,6}]decan. Die Zahnersatzteile eignen sich für die Versorgung mit Kronen, Brücken, Inlays und dergleichen.

EP 0 475 239 A2 betrifft einen polymerisierbaren Dentalwerkstoff auf Basis eines ethylenisch ungesättigten Monomers, der neben einem Katalysator für die Kalt-, Heiß- und/oder Photopolymerisation 20 - 90 Gewichts-% eines anorganischen Füllstoffs aus einer Mischung aus (A) amorphen, kugelförmigen Teilchen aus Siliciumdioxid und bis zu 20 Mol-% eines Oxids mindestens eines Elements der Gruppen I, II, III und IV des Periodensystems mit einem Brechnungsindex von 1,50 bis 1,58 und einer durchschnittlichen Primärteilchengröße von 0,1 bis 1,0 µm und (B) Quarz-, Glaskeramik- oder Glaspulver oder deren Mischungen mit einem Brechungsindex von 1,50 bis 1,58 und einer durchschnittlichen Primärteilchengröße von 0,5 bis 5,0 µm sowie gegebenenfalls geringen Mengen an weiteren Füllstoffen zur Erhöhung der Opazität und Einstellung der Viskosität enthält. Die aus dem Dentalwerkstoff hergestellten Fertigteile zeichnen sich durch ihre gute Transparenz und Polierbarkeit aus.

Aus DE 41 10 612 A1 ist ein Dentalmaterial bekannt, das neben monomeren Dimethacrylaten und einem α-Diketon/Amin-System als Katalysator für die Photopolymerisation ein Füllstoff-Gemisch aus 80 - 90 Gewichts-% Bariumaluminiumsilicatglas mit einer mittleren Teilchengröße von 0,5 - 1,5 Mikrometer und 10 - 20 Gewichts-% Siliciumdioxid mit einer mittleren Teilchengröße von 0,04 - 0,06 Mikrometer enthält. Aus dem Dentalmaterial lassen sich röntgenopake, abrasionsfeste und hochglanzpolierbare Zahnfüllungen und Inlays herstellen.

Befestigungszemente werden benutzt, um Inlays, Onlays, Kronen, Brücken, auch sogenannte Klebe- oder Ahäsivbrücken (Maryland-Brücken), Verblendschalen und dergleichen mit der Zahnsubstanz zu verbinden. Neben infolge von Abbindungsvorgängen härtenden Zementen, wie zum Beispiel den Zinkoxid-Phosphat-Zementen, sind zunehmend auch solche in Gebrauch, die durch Polymerisaion aushärten. Die polymerisierbaren Befestigungszemente enthalten als Monomere üblicherweise Ester der Acrylsäure beziehungsweise Methacrylsäure, meist einen feinteiligen anorganischen Füllstoff und daneben die Polymerisation auslösende Katalysatoren.

Aus EP 0 064 834 B1 ist ein Klebstoff zum Ankleben eines Gegenstandes an einen Zahn bekannt, der ein Bindemittelharz, verdünnendes Monomer, einen anorganischen Füllstoff in einer Menge von mindestens 20 Gewichts-% und einen Photoinitiator zur Auslösung der Polymerisationn bei Bestrahlung mit sichtbarem Licht enthält. Als Photoinitiator wird ein Gemisch aus einem α-Diketon, ausgewählt aus zum Beispiel Campherchinon, Benzil, Biacetyl, 9,10-Phenanthrenchinon und Naphthochinon, und einem Amin, besonders einem Dialkanol- oder Trialkanolamin, eingesetzt. Als Füllstoffe werden anorganische Gläser, wie zum Beispiel Bariumaluminiumsilicatglas und Lithiumaluminiumsilicatglas, bevorzugt.

Nach DE 34 41 564 C2 lassen sich die Metallflächen von Adhäsivbrücken fest, dicht und spaltfrei mit dem Zahnschmelz verbinden, wenn der dafür benutzte Klebstoff neben Methacrylsäureestern und anorganischem Füllstoff aus silanisiertem Siliciumdioxid mit einer Teilchengröße bis zu 0,04 Mikrometer sowohl einen Katalysator für die chemische Kaltpolymerisation (Autopolymerisation) als auch einen Katalysator für die Photopolymerisation enthält.

Aus Schweiz Monatsschr Zahnmed. Vol. 99, 4 /1989, ist ein niedrigviskoser mikrogefüllter Komposit-Zement bekannt, der durch zweistufige Photopolymerisation gehärtet wird und zunächst gelb gefärbt ist und erst durch die Endhärtung seine definitive Farbe erhält. Dieser Zement enthält zwei Initiator-Systeme mit hohem Campherchinon-Anteil für die Photopolymerisation, deren Absorptionsmaxima bei verschiedenen Wellenlängen des sichtbaren Lichtes liegen. Das Anhärten erfolgt mit Licht einer Wellenlänge, die größer als 470 nm ist, das Endhärten mit Licht der Wellenlänge von rund 470 nm. Durch die zunächst von der Zahnfarbe abweichende Farbe des Zements und seine nach dem Anhärten marzipanähnliche Konsistenz läßt sich der Zement gut bearbeiten und ein Überschuß an Zement, ohne daß die Zahnsubstanz beschädigt wird, rasch und sicher entfernen.

Ein ähnlicher Befestigungszement, der zusätzlich zum Katalysator für die Photopolymerisation noch einen Katalysator für die Kaltpolymerisation enthalten kann, ist aus DE 41 10 611 A1 bekannt.

Der Erfindung liegt die Aufgabe zugrunde, ein pastenförmiges, monomere (Meth)Acrylsäureester und feinteiligen anorganischen Füllstoff enthaltendes polymerisierbares Dentalmaterial zu finden, das sowohl zur Herstellung von Zahnrestaurationen, wie Zahnfüllungen, Kronen, Brükken, Verblendungen, Inlays, Onlays und künstlichen Zähnen, als auch zur Befestigung von Zahnrestaurationen und orthodontischen Vorrichtungen an den natürlichen Zähnen und zur Reparatur von dentalkeramischen Teilen geeignet ist. Das Dentalmaterial soll als pastenförmige Masse weich-modellierbar sein und dennoch die Fähigkeit besitzen, die ihm beim Herstellen der Zahnrestaurationen durch Modellieren gegebene Form beizubehalten. Letztere Eigenschaft wird im folgenden als Standfestigkeit bezeichnet. Mangelnde Standfestigkeit bei der Bearbeitung und Verarbeitung herkömmlicher Dentalmaterialien ist ein gravierendes Problem bei der zahntechnischen Herstellung von Zahnrestaurationen. Die aus dem Dentalmaterial durch zahntechnisches Formen und Modellieren und Polymerisation hergestellten Zahnrestaurationen sollen hochglanzpolierbar sein und in ihrem Verschleißverhalten und den optischen und mechanischen Eigenschaften natürlichen Zähnen nahekommen.

Das die Lösung der Aufgabe darstellende Dentalmaterial ist erfindungsgemäß dadurch gekennzeichnet, daß der anorganische Füllstoff aus 5 - 100 Gewichts-% synthetischer kristalliner Kieselsäure mit Schichtstruktur und 0 - 95 Gewichts-% Glas besteht und die Kieselsäure eine Korngrößenverteilung von 1 - 40 Mikrometer und eine mittlere Teilchengröße von etwa 4 Mikrometer und das Glas eine Korngrößenverteilung von 0,1 - 5 Mikrometer und eine mittlere Teilchengröße von etwa 0,7 Mikrometer aufweist. Das Dentalmaterial enthält 20 - 80 Gewichts-%, vorzugweise 40 - 75 Gewichts-%, des anorganischen Füllstoffs.

Besonders bewährte Ausführungsformen des erfindungsgemäßen Dentalmaterials enthalten 40 - 55 Gewichts-% des vollständig aus synthetischer kristalliner Kieselsäure mit Schichtstruktur bestehenden Füllstoffs - diese Ausführungsform wird bevorzugt, wenn das Dentalmaterial als Befestigungszement eingesetzt wird, - oder 60 - 75 Gewichts-% des Füllstoffs aus 5 - 50 Gewichts-%, vorzugsweise 10 - 30 Gewichts-%, synthetischer kristalliner Kieselsäure mit Schichtstruktur und 50 - 95 Gewichts-%, vorzugsweise 70 - 90 Gewichts-%, Glas.

Als Kieselsäure mit Schichtstruktur wird eine der synthetischen Kieselsäuren mit Schichtstruktur, wie sie zum Beispiel aus DE 34 00 130 A1 bekannt sind, eingesetzt. Die Teilchengröße der Kieselsäure und des Glases wird mittels Röntgen-Streulicht-Zentrifuge bestimmt. Kieselsäure und Glas werden vorzugsweise in silanisierter Form, zum Beispiel durch Behandlung mit 3-Methacryloyloxypropyl-trimethoxysilan, benutzt.

Besonders bewährt hat sich das Dentalmaterial, wenn das Glas einen Brechungsindex von 1,46 - 1,53 besitzt und der (Meth)Acrylsäureester-Anteil entsprechend ausgewählt wird.

Vorzugsweise ist das Glas ein Bariumaluminiumborosilicat-Glas und besteht der (Meth)Acrylsäureester-Anteil aus Monomer-Mischungen, die einen Brechungsindex von 1,49 - 1,50 und eine Viskosität von 1500 - 4000 mPa s bei 23° C aufweisen.

Für den (Msth)Acrylsäureester-Antell geeignete Monomer-Mischungen lassen sich aus den folgenden an sich bekannten monomeren Di- und Poly(meth)acrylaten auswählen:
Diurethandi(meth)acrylat aus 2,2,4-Trimethylhexamethylendiisocyanat und 2-Hydroxyethyl(meth)acrylat,
Diurethandi(meth)acrylat aus Bis-(diisocyanatomethyl)-tricyclodecan und 2-Hydroxyethyl(meth)acrylat,
Decandioldi(meth)acrylat,
Dodecandioldi(meth)acrylat,
Triethylenglykoldi(meth)acrylat,
Bis-[4-(2-hydroxy-3-methacryloyloxypropoxy)-phenyl]-dimethylmethan,
Bis-[4-(2-hydroxy-3-acryloyloxypropoxy)-phehyl]-dimethylmethan,
Tri(meth)acryloyloxyethoxytrimethylolpropan,
Tetra(meth)acryloyloxyethoxypentaerythrit,
Tetra(meth)acryloyloxyisopropoxypentaerythrit und
Hexa(meth)acryloyloxyethoxydipentaerythrit.

Es hat sich gezeigt, daß sich ein zu 5 - 60 Gewichts-% aus monomerem Poly(meth)acrylat bestehender (Meth)Acrylsäureester-Anteil vorteilhaft auf die Abrasionsfestigkeit und die mechanischen Eigenschaften der Zahnrestaurationen auswirkt. Bevorzugt wird ein (Meth)Acrylsäureester-Anteil, der zu 30 - 60 Gewichts-% aus monomerem Poly(meth)acrylat besteht.

Das Dentalmaterial kann sowohl durch Heißpolymerisation oder Kaltpolymerisation als auch durch Photopolymerisation ausgehärtet werden.

Geeignete Katalysatoren für die Heißpolymerisation sind zum Beispiel organische Peroxide, wie Dibenzoylperoxid, für die Kaltpolymerisation zum Beispiel die Redox-Systeme, vorzugsweise solche aus organischen Peroxiden und Aminen, und für die Photopolymerisation Keton/Amin-Systeme, wie sie aus GB 1 408 265 B1 bekannt sind, zum Beispiel Campherchinon/Amin.

Die Aushärtung des Dentalmaterials durch Photopolymerisation wird bevorzugt. Es enthält dann 0,1 - 0,5 Gewichts-%, vorzugsweise 0,1 - 0,3 Gewichts-%, eines Keton/Amin-Systems, wobei sich als Amine N,N-Dimethyl-p-toluidin, N,N-Bis-(2-hydroxyethyl)-p-toluidin und Ester der 4-Dimethylaminobenzoesäure, wie der Ethyl- und der Butoxyethylester, besonders bewährt haben. Dem Dentalmaterial kann als weiterer photoaktiver Bestandteil zusätzlich ein Benzilacetal, vorzugsweise in einer Menge von 0,02 - 0,1 Gewichts-%, zugefügt werden.

Für den Gebrauch hat sich die Formulierung des nur Katalysatoren für die Photopolymerisation enthaltenden Dentalmaterials als lagerfähiges Einkomponenten-Material in Pasten-Form bewährt. Ein zusätzlich durch Kaltpolymerisation auszuhärtendes Dentalmaterial liegt vorzugsweise als pastenförmiges Zweikomponenten-Material vor, wobei zweckmäßgerweise die Zusammensetzung der einen Paste der des Einkomponenten-Materials entspricht und die andere Paste neben (Meth)Acrylsäureester und anorganischem Füllstoff den Katalysator für die Kaltpolymerisation, das Peroxid, enthält.

Im gebrauchsfertigen Dentalmaterial sind außerdem noch Farbpigmente, Antioxidationsmittel, Stabilisatoren und andere übliche Zusatzstoffe vorhanden.

In herkömmlichen Dentalmaterialien werden pyrogene oder gefällte Kieselsäuren aufgrund ihrer Feinteiligkeit eingesetzt, um hochglanzpolierbare Materialien zu erhalten und um nach dem Prinzip der dichtesten Kugelpackung einen möglichst hohen Füllstoff-Gehalt zu erreichen. Um Standfestigkeit zu erlangen, muß ein Füllstoff-Gehalt zwischen Geschmeidigkeit und Trockenheit der Dentalmaterialien angestrebt werden. Die Dentalmaterialien sind dann sehr fest und daraus erhaltene ausgehärtete Produkte hart und spröde, da der Monomerfilm um die Füllstoffteilchen sehr klein gehalten werden muß. Versucht man auf diesem Weg, weichere Pasten zuzubereiten, können sowohl die feinteiligen, kugelähnlichen Kieselsäureteilchen als auch die sich wie Kugeln verhaltenden feinstgemahlenen Glasteilchen aneinander vorbeigleiten, und es kommt zum beim zahntechnischen Modellieren unerwünschten Fließen daraus hergestellter Strukturen und Formen.

Überraschenderweise lassen sich durch den Einsatz synthetischer kristalliner Kieselsäuren mit Schichtstruktur in einer Korngrößenverteilung von 1 - 40 Mikrometer (Komgrößenverteilung von feinteiligen, pyrogenen oder gefällten Kieselsäuren: 0,01 - 0,4 Mikrometer) aufgrund ihrer makroporigen Schichtstruktur und ihrer blättrigen Oberflächenmorphologie sowohl die rheologischen als auch die optischen und mechanischen Zielkonflikte herkömmlicher Dentalmaterialien lösen. Das pastenförmige Dentalmaterial gemäß der Erfindung zeichnet sich durch seine weiche bis weich-knetbare, nicht-klebrige Konsistenz aus; auch bei längerem modellierendem Bearbeiten behält es seine Standfestigkeit. Es läßt sich gleichmäßig verteilen, sehr dünn ausstreichen, ohne trocken zu werden und zu reißen, sowie gut formen und modellieren und zeigt, sobald die scherend-mechanische Beanspruchung aufhört, wieder die vorige Standfestigkeit. Aus dem Dentalmaterial modellierte und noch nicht durch Polymerisation ausgehärtete Formen behalten ihren Zustand; auch feinste Details der Modellation zerfließen nicht. Als Zement zur Befestigung von Zahnrestaurationen und orthodontischen Vorrichtungen an damit zu versorgenden Zähnen verteilt es sich leicht und gleichmäßig im Spalt zwischen den miteinander zu verbindenden Oberflächen. Ein aus dem Spalt austretender Überschuß an Zement fließt nicht weg, sondern verharrt nach Abbau der Scherspannung am Ort des Austritts und läßt sich leicht entfernen.

In dem erfindungsgemäßen Dentalmaterial kann der Füllstoff-Gehalt niedriger gehalten werden als in herkömmlichen Dentalmaterialien, so daß mehr verbindendes Polymer die Füllstoffteilchen besser zusammenhält, wodurch das Dentalmaterial biegsamer und zäher wird. Gleichzeitig bewirkt die makroporige Schichtstruktur eine Durchdringung der Kieselsäure mit Monomer beziehungsweise Polymer, so daß die Füllstoffteilchen - anders als homogene (d. h. keine Schichtstruktur aufweisende) 1 - 40 Mikrometer große Füllstoffteilchen - die Hochglanzpolierbarkeit nicht beeinträchtigen. Das bedeutet, daß das erfindungsgemäße Dentalmaterial einen geringeren Füllstoff-Gehalt benötigt, um nicht nur zeitweise, sondern dauerhaft standfest zu sein, und weich-modellierbar eingestellt werden kann und daß die daraus hergestellten Zahnrestaurationen zäh-mechanische Eigenschaften (hohe Biegefestigkeit bei mäßig hohem Biegemodul) aufweisen und trotz der "Grobheit" der Kieselsäure hochglanzpolierbar sind. Aufgrund der zäh-mechanischen Eigenschaften der Zahnrestaurationen wird eine mit der des natürlichen Zahnschmelzes vergleichbare, hohe Abrasionsfestigkeit gegenüber Kontaktreibung erzielt. Infolge des hohen Polymer-Anteils und der durch die monomeren Poly(meth)acrylate bedingten hohen Vernetzung verbleiben die Füllstoffteilchen deutlich länger in der dem Verschleiß ausgesetzten Oberfläche der Zahnrestaurationen. Bei der Kontaktreibung werden die feineren, harten und unelastischen Glasteilchen durch die"gröberen", polymer-durchdrungenen, elastisch nachgebenden Teilchen der Kieselsäure mit Schichtstruktur geschützt.

Die aus dem erfindungsgemäßen Dentalmaterial durch Polymerisation erhaltenen ausgehärteten Zahnrestaurationen zeichnen sich durch zäh-mechanische Eigenschaften aus; speziell im Hinblick auf die Schlagzähigkeit, die Biegefestigkeit und den Biegemodul sind sie den natürlichen Zähnen ähnlich. Außerdem wird eine der des Zahnschmelzes entsprechende Abrasionsfestigkeit erreicht. Die Zahnrestaurationen sind hochglanzpolierbar. Es können der Transparenz natürlicher Zähne ähnliche Transparenzeigenschaften eingestellt werden, da die Transparenz des keine Pigmente enthaltenden Dentalmaterials bei über 70% auf 1 mm Schichtstärke liegt (Zahnschmelz 70 - 80% auf 1 mm). Die Zahnrestaurationen weisen ein ästhetisch ansprechendes Aussehen und einen hohen Kaukomfort auf und sind dauerhaft belastbar.

Das erfindungsgemäße Dentalmaterial eignet sich besonders zur Herstellung von Zahnfüllungen, Kronen, Brücken, Verblendungen, Inlays, Onlays und künstlichen Zähnen und als Befestigungszement. Es kann auch für die Reparatur beschädigter dentalkeramischer Teile eingesetzt werden. Möglich ist auch seine Anwendung als Reparaturmaterial für technische Keramik.

Zur näheren Erläuterung werden in den folgenden Beispielen für das erfindungsgemäße Dentalmaterial geeignete Monomer-Mischungen (Beispiele 1 - 7), beispielhafte Ausführungsformen des Dentalmaterials (Beispiele 8 - 15) und die Herstellung von Prüfkörpern daraus beschrieben. Die Eigenschaften der Prüfkörper werden bestimmt und mit denen von Prüfkörpern aus bekannten polymerisierbaren Dentalmaterialien vom Komposit-Typ (Feinhybrid) und aus Vollkeramik (Glas- und Feldspat-Basis) verglichen. In den erfindungsgemäßen Ausführungsformen werden als anorganische Füllstoffe eine synthetische kristalline Kieselsäure mit Schichtstruktur, die eine spezifische Oberfläche von 50 - 60 m²/g und einen Brechungsindex von 1,43 aufweist, und ein feinstgemahlenes Bariumaluminiumborosilicat-Glas aus 55 Gewichts-% SiO₂, 25 Gewichts-% BaO, 10 Gewichts-% Al₂O₃ und 10 Gewichts-% B₂O₃ mit einem Brechungsindex von 1,53 eingesetzt.

### Beispiele 1 - 7

Es werden 7 Monomer-Mischungen der in der Tabelle I angegebenen Zusammensetzung hergestellt. Tabelle II enthält den Brechungsindex und die Viskosität (23° C) der Mischungen.

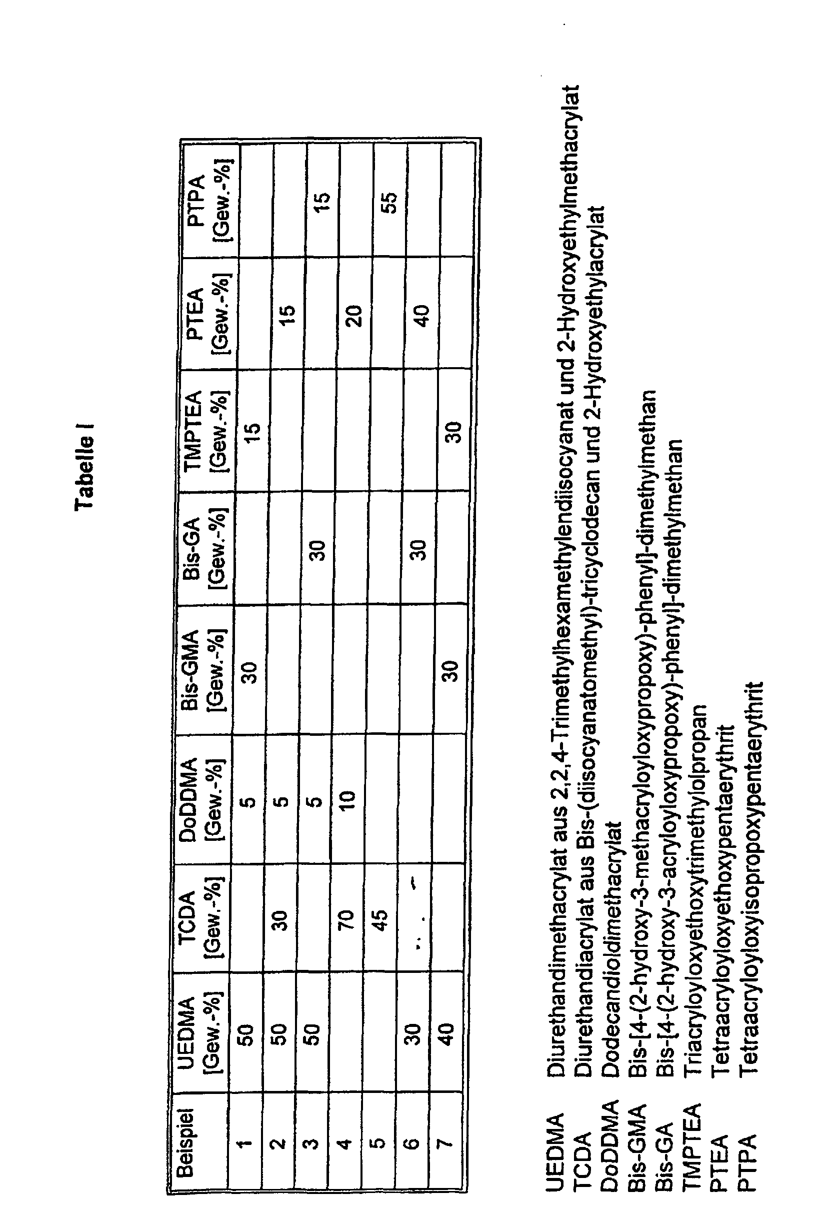

**Tabelle II**

| Beispiel | Brechungsindex | Viskosität [m Pa s] |
|---|---|---|
| 1 | 1,497 | 2500 |
| 2 | 1,490 | 2100 |
| 3 | 1,499 | 2600 |
| 4 | 1,494 | 2900 |
| 5 | 1,491 | 3500 |
| 6 | 1,499 | 2800 |
| 7 | 1,500 | 3200 |

### Beispiel 8

| Befestigungszement (Komposit-Zement) aus | Gewichts-% |
|---|---|
| Monomer-Mischung nach Beispiel 2 | 51,68 |
| Kieselsäure mit Schichtstruktur¹, mittlere Teilchengröße 4 Mikrometer | 48,00 |
| Phenanthrenchinon | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |

### Beispiel 9

Durch Photopolymerisation und Kaltpolymerisation auszuhärtender Befestigungszement in Form zweier Pasten

| Paste A | Gewichts-% |
|---|---|
| Monomer-Mischung nach Beispiel 2 | 53,8 |
| Kieselsäure mit Schichtstruktur¹, mittlere Teilchengröße 4 Mikrometer | 45,0 |
| Dibenzoylperoxid | 1,0 |
| 2,6-Di-tert.-butyl-4-methylphenol | 0,2 |

| Paste B | |
|---|---|
| Monomer-Mischung nach Beispiel 4 | 49,48 |
| Kieselsäure mit Schichtstruktur¹, mittlere Teilchengröße 4 Mikrometer | 50,00 |
| Phenanthrenchinon | 0,2 |
| N,N-Dimethyl-p-toluidin | 0,3 |
| 2,6-Di-tert.-butyl-4-methylphenol | 0,02 |

| | |
|---|---|
| ¹silanisiert mit 3-Methacryloyloxypropyltrimethoxysilan | |

### Beispiel 10

| Dentalmaterial aus | Gewichts-% |
|---|---|
| Monomer-Mischung nach Beispiel 1 | 26,58 |
| Bariumaluminiumborosilicat-Glas¹, | |
| mittlere Teilchengröße 0,7 Mikrometer | 58,5 |
| Kieselsäure mit Schichtstruktur¹, | |
| mittlere Teilchengröße 4 Mikrometer | 14,5 |
| Campherchinon | 0,1 |
| Benzildimethylacetal² | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |
| ²1,2-Diphenyl-2,2-dimethoxyethanon | |

### Beispiel 11

| Dentalmaterial aus | Gewichts-% |
|---|---|
| Monomer-Mischung nach Beispiel 2 | 27,08 |
| Bariumaluminiumborosilicat-Glas¹, mittlere Teilchengröße 0,7 Mikrometer | 58,0 |
| Kieselsäure mit Schichtstruktur¹, mittlere Teilchengröße 4 Mikrometer | 14,5 |
| Campherchinon | 0,1 |
| Benzildimethylacetal² | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |
| ²1,2-Diphenyl-2,2-dimethoxyethanon | |

### Beispiel 12

| Dentalmaterial aus | Gewichts-% |
|---|---|
| Monomer-Mischung nach Beispiel 3 | 27,08 |
| Bariumaluminiumborosilicat-Glas¹, mittlere Teilchengröße 0,7 Mikrometer | 58,0 |
| Kieselsäure mit Schichtstruktur¹, mittlere Teilchengröße 4 Mikrometer | 14,5 |
| Campherchinon | 0,1 |
| Benzildimethylacetal² | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |
| ²1,2-Diphenyl-2,2-dimethoxyethanon | |

### Beispiel 13

| Dentalmaterial aus | Gewichts-% |
|---|---|
| Monomer-Mischung nach Beispiel 4 | 27,08 |
| Bariumaluminiumborosilicat-Glas¹, mittlere Teilchengröße 0,7 Mikrometer | 58,0 |
| Kieselsäure mit Schichtstruktur¹, mittlere Teilchengröße 4 Mikrometer | 14,5 |
| Campherchinon | 0,1 |
| Benzildimethylacetal² | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |
| ²1,2-Diphenyl-2,2-dimethoxyethanon | |

### Beispiel 14

| Dentalmaterial aus | Gewichts-% |
|---|---|
| Monomer-Mischung nach Beispiel 1 | 27,58 |
| Bariumaluminiumborosilicat-Glas¹, mittlere Teilchengröße 0,7 Mikrometer | 59,0 |
| Kieselsäure mit Schichtstruktur¹, mittlere Teilchengröße 4 Mikrometer | 13,0 |
| Campherchinon | 0,1 |
| Benzildimethylacetal² | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |
| ²1,2-Diphenyl-2,2-dimethoxyethanon | |

### Beispiel 15

| Dentalmaterial aus | Gewichts-% |
|---|---|
| Monomer-Mischung nach Beispiel 1 | 28,58 |
| Bariumaluminiumborosilicat-Glas¹, mittlere Teilchengröße 0,7 Mikrometer | 60,0 |
| Kieselsäure mit Schichtstruktur¹, mittlere Teilchengröße 4 Mikrometer | 11,0 |
| Campherchinon | 0,1 |
| Benzildimethylacetal² | 0,1 |
| N,N-Dimethyl-p-toluidin | 0,2 |
| 2,6-Di-tert.-butyl-4-methylphenol | 0,02 |

| | |
|---|---|
| ¹silanisiert mit 3-Methacryloyloxypropyltrimethoxysilan | |
| ²1,2-Diphenyl-2,2-dimethoxyethanon | |

### Herstellung der Prüfkörper

Proben des in den Beispielen 10 - 15 beschriebenen Dentalmaterials werden in offene Hohlformen aus Metall gegeben und 180 beziehungsweise 360 Sekunden lang in einem Licht-Polymerisationsgerät, wie es für das Aushärten von photopolymerisierbarem Kronen- und Brückenmaterial bekannt ist (Dentacolor XS der Firma Heraeus Kulzer GmbH, Deutschland), bestrahlt. Die so erhaltenen Prüfkörper werden für die Bestimmung von Transparenz, Abrasionsfestigkeit, Schlagzähigkeit, Biegefestigkeit und Biegemodul eingesetzt.

### Bestimmung von Transparenz, Abrasionsfestigkeit, Schlagzähigkeit, Biegefestigkeit und Biegemodul

Für die Bestimmung der Transparenz werden nach ISO 10 477 20 (Durchmesser) x 1 mm große Prüfkörper und ein Farbmeßgerät benutzt. Es werden die Farbwerte über schwarzem und weißem Untergrund gemessen; die Differenz dieser Werte dient als Maß für die Transparenz.

Die Abrasionsfestigkeit wird durch Verschleißmessungen mit dem in Schweiz Monatsschr Zahnmed. Vol. 100 (1990), 953 - 960, beschriebenen Kausimulator bestimmt. Für die Verschleißmessungen werden Prüfkörper mit einem Durchmesser von 10 mm und einer Dicke von 2 mm, die durch 180 Sekunden langes Bestrahlen und Polieren mit Siliciumcarbid-Schleifpapier hergestellt werden, und ein Keramik-Stäbchen als Gegenzahnstempel benutzt.

Die Schlagzähigkeit wird nach DIN 53 453 an 15 x 10 x 3 mm großen Prüfkörpern, die Biegefestigkeit und der Biegemodul nach ISO 10 477 an 25 x 2 x 2 mm großen Prüfkörpem im Dreipunktbiegetest ermittelt.

Die erhaltenen Werte für Transparenz, Abrasionsfestigkeit, Schlagzähigkeit, Biegefestigkeit und Biegemodul und - zum Vergleich dazu - die entsprechenden Werte bekannter Dentalmaterialien (Feinhybrid, Vollkeramik) werden zusammen mit einigen für Zahnschmelz und Dentin bekannten Werten in der Tabelle III angegeben.

**Tabelle III**

| Prüfkörper | Transparenz [%] | Abrasionsfestigkeit [µm] | Schlagzähigkeit [KJ/m²] | Biegefestigkeit [MPa] | Biegemodul [MPa] |
|---|---|---|---|---|---|
| Zahnschmelz | 70 - 80 | 40 - 60 | - | 10 - 15 | 80 - 90 000 |
| Dentin | 60 - 70 | - | - | 40 - 60 | 13 - 18 000 |
| Beispiel 10 | 70 | 70 + 10 | 2,5 | 120 | 9300 |
| Beispiel 11 | 73 | 50 + 15 | 2,8 | 120 | 9700 |
| Beispiel 12 | 69 | 60 + 12 | 3,2 | 125 | 9500 |
| Beispiel 13 | 71 | 45 + 13 | 3,5 | 135 | 8900 |
| Beispiel 14 | 70 | 60 + 15 | 2,8 | 134 | 8500 |
| Beispiel 15 | 71 | 55 + 13 | 3,1 | 110 | 8300 |
| Feinhybrid (Vgl.) | 40 - 50 | 90 - 120 | 1,5 - 2,0 | 120 - 200 | 13 - 20 000 |
| Vollkeramik (Vgl.) | 70 - 80 | 40 - 60 | 1 - 1,5 | 80 - 120 | 80 - 120 000 |

## Patentansprüche

1. Polymerisierbares Dentalmaterial, das monomere (Meth)Acrylsäureester, feinteiligen anorganischen Füllstoff und einen Polymerisationskatalysator enthält, **dadurch gekennzeichnet, daß** der anorganische Füllstoff aus 5 - 100 Gewichts-% synthetischer kristalliner Kieselsäure mit Schichtstruktur und 0 - 95 Gewichts-% Glas besteht und die Kieselsäure eine Komgrößenverteilung von 1 - 40 Mikrometer und eine mittlere Teilchengröße von etwa 4 Mikrometer und das Glas eine Komgrößenverteilung von 0,1 - 5 Mikrometer und eine mittlere Teilchengröße von etwa 0,7 Mikrometer aufweist.

2. Dentalmaterial nach Anspruch 1, **dadurch gekennzeichnet, daß** es 20 - 80 Gewichts-% des anorganischen Füllstoffs enthält.

3. Dentalmaterial nach Anspruch 1 oder 2, **dadurch gekennzeichnet, daß** es 40 - 75 Gewichts-% des anorganischen Füllstoffs enthält.

4. Dentalmaterial nach einem der Ansprüche 1 bis 3, **dadurch gekennzeichnet, daß** der anorganische Füllstoff zu 40 - 55 Gewichts-% darin enthalten ist und vollständig aus der Kieselsäure besteht.

5. Dentalmaterial nach einem der Ansprüche 1 bis 3, **dadurch gekennzeichnet, daß** der anorganische Füllstoff zu 60 - 75 Gewichts-% darin enthalten ist und aus einem Gemisch aus 5 - 50 Gewichts-% der Kieselsäure und 50 - 95 Gewichts-% des Glases besteht.

6. Dentalmaterial nach Anspruch 5, **dadurch gekennzeichnet, daß** das Gemisch aus 10 - 30 Gewichts-% der Kieselsäure und 70 - 90 Gewichts-% des Glases besteht.

7. Dentalmaterial nach Anspruch 1, 2, 3, 5 oder 6, **dadurch gekennzeichnet, daß** das Glas einen Brechungsindex von 1,46 - 1,53 aufweist.

8. Dentalmaterial nach Anspruch 1, 2, 3, 5, 6 oder 9 **dadurch gekennzeichnet, daß** das Glas ein Bariumaluminiumborosilicat-Glas ist.

9. Dentalmaterial nach einem der Ansprüche 1 bis 8, **dadurch gekennzeichnet, daß** die Kieselsäure und das Glas silanisiert sind.

10. Dentalmaterial nach einem der Ansprüche 1 bis 9, **dadurch gekennzeichnet, daß** es als monomere (Meth)Acrylsäureester mindestens ein monomeres Di(meth)acrylat aus der Gruppe Diurethandi(meth)acrylat aus 2,2,4-Trimethylhexamethylendiisocyanat und 2-Hydroxyethyl(meth)acrylat, Diurethandi(meth)acrylat aus Bis-(diisocyanatomethyl)-tricyclodecan und 2-Hydroxyethyl(meth)acrylat, Decandioldi(meth)acrylat, Dodecandioldi(meth)acrylat, Triethylenglykoldi(meth)acrylat, Bis-[4-(2-hydroxy-3-methacryloxypropoxy)-phenyl]-dimethylmethan und Bis-[4-(2-hydroxy-3-acryloyloxypropoxy)phenyl]-dimethylmethan und mindestens ein monomeres Poly(meth)acrylat aus der Gruppe Tri(meth)acryloyloxyethoxytrimethylolpropan, Tetra(meth)acryloyloxyethoxypentaerythrit, Tetra(meth)acryloyloxyisopropoxypentaerythrit und Hexa(meth)acryloyloxyethoxydipentaerythrit enthält.

11. Dentalmaterial nach Anspruch 10, **dadurch gekennzeichnet, daß** die monomeren (Meth)-Acrylsäureester zu 5 - 60 Gewichts-% aus monomerem Poly(meth)acrylat bestehen.

12. Dentalmaterial nach Anspruch 11, **dadurch gekennzeichnet, daß** die monomeren (Meth)Acrylsäureester zu 30 - 60 Gewichts-% aus monomerem Poly(meth)acrylat bestehen.

13. Verwendung des Dentalmaterials nach einem der Ansprüche 1 - 12 zur Herstellung von Zahnfüllungen, Kronen, Brücken, Verblendungen, Inlays, Onlays und künstlichen Zähnen, als Befestigungszement, zur Reparatur von dentalkeramischen Teilen und als sonstiges Material für prothetische, konservierende und präventive Zahnbehandlungen.

## Revendications

1. Matériau dentaire polymérisable qui contient des esters d'acide (méth)acrylique monomères, une charge inorganique finement divisée et un catalyseur de polymérisation, **caractérisé en ce que** la charge inorganique consiste en 5-100 % en masse d'acide silicique cristallin synthétique à structure feuilletée et en 0-95 % en masse de verre et l'acide silicique présente une répartition granulométrique de 1-40 µm et une taille de particules moyenne d'environ 4 µm et le verre présente une répartition granulométrique de 0,1-5 µm et une taille de particules moyenne d'environ 0,7 µm.

2. Matériau dentaire selon la revendication 1 **caractérisé en ce qu'**il contient 20-80 % en masse de charge inorganique.

3. Matériau dentaire selon la revendication 1 ou 2 **caractérisé en ce qu'**il contient 40-75 % en masse de charge inorganique.

4. Matériau dentaire selon l'une des revendications 1 à 3 **caractérisé en ce que** la charge inorganique y est contenue à raison de 40-55 % en masse et consiste totalement en acide silicique.

5. Matériau dentaire selon l'une des revendications 1 à 3 **caractérisé en ce que** la charge inorganique y est contenue à raison de 60-75 % en masse et consiste en un mélange de 5-50 % en masse d'acide silicique et 50-95 % en masse de verre.

6. Matériau dentaire selon la revendication 5 **caractérisé en ce que** le mélange consiste en 10-30 % en masse d'acide silicique et 70-90 % en masse de verre.

7. Matériau dentaire selon la revendication 1, 2, 3, 5 ou 6 **caractérisé en ce que** le verre présente un indice de réfraction de 1,46-1,53.

8. Matériau dentaire selon la revendication 1, 2, 3, 5, 6 ou 9 **caractérisé en ce que** le verre est un verre de borosilicate de baryum et d'aluminium.

9. Matériau dentaire selon l'une des revendications 1 à 8 **caractérisé en ce que** l'acide silicique et le verre sont silanisés.

10. Matériau dentaire selon l'une des revendications 1 à 9 **caractérisé en ce qu'**il contient comme esters d'acide (méth)acrylique monomères au moins un di(méth)acrylate monomère du groupe du di(méth)acrylate de diuréthane issu du 2,2,4-triméthylhexaméthylène-diisocyanate et du (méth)acrylate de 2-hydroxyéthyle, du di(méth)acrylate de diuréthane issu du bis-(diisocyanatométhyl)tricyclodécane et du (méth)acrylate de 2-hydroxyéthyle, du di(méth)acrylate de décanediol, du di(méth)acrylate de dodécanediol, du di(méth)acrylate de triéthylèneglycol, du bis-[4-(2-hydroxy-3-méthacryloxypropoxy)-phényl]-diméthylméthane et du bis-[4-(2-hydroxy-3-acryloyloxypropoxy)phényl]-diméthylméthane et au moins un poly(méth)acrylate monomère du groupe du tri(méth)acryloyloxyéthoxytriméthylolpropane, du tétra(méth)-acryloyloxyéthoxypentaérythritol, du tétra(méth)acryloyloxyisopropoxy-pentaérythritol et de l'hexa(méth)acryloyloxyéthoxydipentaérythritol.

11. Matériau dentaire selon la revendication 10 **caractérisé en ce que** les esters d'acide (méth)acrylique monomères consistent à raison de 5-60 % en masse en poly(méth)acrylate monomère.

12. Matériau dentaire selon la revendication 11 **caractérisé en ce que** les esters d'acide (méth)acrylique monomères consistent à raison de 30-60 % en masse en poly(méth)acrylate monomère.

13. Utilisation du matériau dentaire selon l'une des revendications 1 à 12 pour la production d'obturations dentaires, de couronnes, de bridges, de revêtements, d'incrustations en profondeur, d'incrustations de surface et de dents artificielles, comme ciment de fixation, pour la réparation de pièces céramiques dentaires et comme autre matériau pour des traitements dentaires prothétiques, conservateurs et préventifs.

## Claims

1. Polymerizable dental material which contains monomeric (meth)acrylic esters, a finely divided inorganic filler and a polymerization catalyst, **characterized in that** the inorganic filler consists of 5 - 100% by weight of synthetic crystalline silica having a layer structure and 0 - 95% by weight of glass and the silica has a particle size distribution of 1-40 micrometers and a mean particle size of about 4 micrometers and the glass has a particle size distribution of 0.1 - 5 micrometers and a mean particle size of about 0.7 micrometers.

2. Dental material according to Claim 1, **characterized in that** it contains 20 - 80% by weight of the inorganic filler.

3. Dental material according to Claim 1 or 2, **characterized in that** it contains 40 - 75% by weight of the inorganic filler.

4. Dental material according to any of Claims 1 to 3, **characterized in that** the inorganic filler is contained therein in an amount of 40 - 55% by weight and consists completely of silica.

5. Dental material according to any of Claims 1 to 3, **characterized in that** the inorganic filler is contained therein in an amount of 60 - 75% by weight and consists of a mixture of 5 - 50% by weight of silica and 50 - 95% by weight of glass.

6. Dental material according to Claim 5, **characterized in that** the mixture consists of 10 - 30% by weight of silica and 70 - 90% by weight of glass.

7. Dental material according to Claim 1, 2, 3, 5 or 6, **characterized in that** the glass has a refractive index of 1.46 - 1.53.

8. Dental material according to Claim 1, 2, 3, 5, 6 or 9, **characterized in that** the glass is a barium aluminium borosilicate glass.

9. Dental material according to any of Claims 1 to 8, **characterized in that** the silica and the glass have been silanized.

10. Dental material according to any of Claims 1 to 9, **characterized in that** it contains, as monomeric (meth)acrylic esters, at least one monomeric di(meth)acrylate from the group consisting of diurethane di(meth)acrylate obtained from 2,2,4-trimethylhexamethylene diisocyanate and 2-hydroxyethyl (meth)acrylate, diurethane di(meth)acrylate obtained from bis(diisocyanatomethyl)tricyclodecane and 2-hydroxyethyl (meth)acrylate, decanediol di(meth)acrylate, dodecanediol di(meth)acrylate, triethylene glycol di(meth)acrylate, bis[4-(2-hydroxy-3-methacryloyloxypropoxy)phenyl]dimethylmethane and bis[4-(2-hydroxy-3-acryloyloxypropoxy)phenyl]dimethyl-methane and at least one monomeric poly(meth)acrylate from the group consisting of tri-(meth)acryloyloxyethoxytrimethylolpropane, tetra-(meth)acryloyloxyethoxypentaerythritol, tetra-(meth)acryloyloxyisopropylpentaerythritol and hexa-(meth)acryloyloxyethoxydipentaerythritol.

11. Dental material according to Claim 10, **characterized in that** the monomeric (meth)acrylic esters comprise 5 - 60% by weight of monomeric poly(meth)acrylate.

12. Dental material according to Claim 11, **characterized in that** the monomeric (meth)acrylic esters comprise 30 - 60% by weight of monomeric poly(meth)acrylate.

13. Use of the dental material according to any of Claims 1 - 12 for the production of dental fillings, crowns, bridges, veneers, inlays, onlays and artificial teeth, as fixing cement, for the repair of dental ceramic parts and as other material for prosthetic, conservative and preventative dental treatments.
